# EUROPÄISCHE PATENTANMELDUNG

(11) **EP 4 083 188 A1**
(43) Veröffentlichungstag der Anmeldung: **02.11.2022**
(21) Anmeldenummer: 22164113.7
(22) Anmeldetag: 24.03.2022
(51) Int. Cl.: C12N 1/20

(54) **BAKTERIELLES BIOPRÄPARAT, VERFAHREN ZU SEINER HERSTELLUNG UND VERWENDUNG**

(30) Priorität: 06.04.2021 DE 102021108535
(71) Anmelder: Geofert LLC, 0162 Tbilisi (GE)
(72) Erfinder: Gvazava, Tengiz, 0136 Tbilisi (GE); Gvazava, Zaal, 0136 Tbilisi (GE); Khurtsidze, Tamar, 0136 Tbilisi (GE); Chanturia, Irakli, 0136 Tbilisi (GE)
(74) Vertreter: Sloboshanin, Sergej

(57) **Zusammenfassung**

Die Erfindung betrifft die Biotechnologie und Mikrobiologie, und zwar ein bakterielles Biopräparat, ein Verfahren zur Herstellung eines bakteriellen Biopräparates, insbesondere ein konzentriertes flüssiges oder pastöses Biopräparat, wobei das Nährmedium für Bakterien ein alkalischer Extrakt aus organischen Substanzen pflanzlichen oder natürlichen Ursprungs aus Kaustobiolithen ist. Die Erfindung bezieht sich auch auf die Verwendung des bakteriellen Biopräparates als Düngemittel für die Blatt- und Wurzeldüngung von Pflanzen, als Fungizid, als Futtermittelzusatz für Tiere oder Vögel, als Präparat zur Förderung der Methanogenese von Biogas, als Stimulator des Pflanzenwachstums.

## Beschreibung

Die Erfindung betrifft die Biotechnologie und Mikrobiologie, und zwar ein bakterielles Biopräparat und ein Verfahren zur Herstellung eines bakteriellen Biopräparats. Insbesondere betrifft die vorliegende Erfindung ein konzentriertes flüssiges oder pastöses Biopräparat, wobei das Kulturmedium für die Bakterien ein alkalischer Extrakt organischer Substanzen pflanzlichen oder natürlichen Ursprungs aus Kaustobiolithen ist. Die Erfindung betrifft auch die Verwendung des bakteriellen Biopräparates als Düngemittel für die Blatt- und Wurzeldüngung von Pflanzen, als Fungizid, als Futtermittelzusatz für Tiere oder Vögel, als Biopräparat zur Förderung der Methanogenese bei der Biogasproduktion, als Stimulator des Pflanzenwachstums. Die Grundlage des bakteriellen Biopräparates ist ein Nährmedium für das Wachstum von Mikroorganismen.

In der Mikrobiologie werden Nährmedien in natürliche, halbsynthetische und synthetische Nährmedien unterteilt.

Natürliche Nährmedien sind solche, die aus natürlichen Materialien hergestellt werden: Blut, Fleisch, Proteine, tierische Organe, Pflanzenextrakte und Pflanzenrohstoffe. Beispiele für solche Medien sind Fleischbrühe, Milchmolke, Bierwürze, Heuaufguss, Agar-Agar, Blut, Gallenflüssigkeit. Unter natürlichen Medien versteht man Medien mit einer unbestimmten Zusammensetzung, die zu verschiedenen Zeiten unterschiedliche Mengen verschiedener Komponente aufweisen können.

Halbsynthetische Medien sind ebenfalls Medien mit einer unbestimmten Zusammensetzung. Sie werden auf der Basis von Naturprodukten unter Zusatz von Substanzen hergestellt, die die Vermehrung von Bakterienkulturen fördern. Zum Beispiel werden Kulturen für die Herstellung von Vitaminen, Aminosäuren und Antibiotika auf halbsynthetischen Medien gezüchtet.

Synthetische Medien werden aus Inhaltsstoffen einer bekannten Zusammensetzung, in bekannten Konzentrationen und Verhältnissen hergestellt, deswegen gehören diese Medien zu Medien mit einer definierten Zusammensetzung.

Das Nährmedium gemäß der vorliegenden Erfindung ist halbsynthetisch. Dieses Medium wird auf der Basis eines Extraktes aus zerkleinerten Kaustobiolithen hergestellt.

Das häufigste natürliche Medium ist Fleisch-Pepton-Brühe, die durch Zugabe von 1,0% Pepton und 0,5% Natriumchlorid zur Fleischbrühe hergestellt wird, wobei der pH-Wert des Mediums durch Zugabe von Alkali auf 7,6 - 7,8 pH eingestellt wird. Falls erforderlich werden zur Stabilisierung des pH-Wertes des Mediums Phosphatpuffermischungen in die Brühe gegeben und anschließend wird das Medium für 35-45 Minuten gekocht bis eine Ausfällung erfolgt. Danach wird das Medium filtriert, mit Wasser bis zum Ausgangsvolumen aufgefüllt und in einem Autoklaven bei 120°C für 15-20 Minuten sterilisiert. Die Basis dieses Mediums ist ein teurer Lebensmittelrohstoff - mageres hochwertiges Kalb- oder Rindfleisch (Spezielle medizinische Mikrobiologie mit der Technik der mikrobiologischen Untersuchung / herausgegeben von A.S. Labinskaya, P.P. Blinkova - M. Medicina 2005, S.480). Dieses Medium wird in der Regel nur bei Laboruntersuchungen eingesetzt. In der industriellen Großproduktion ist die Verwendung dieses Mediums wirtschaftlich unrentabel.

Ebenfalls bekannt im Stand der Technik ist das Nährmedium Luria (LB-Medium) (Wikipedia, https://ru.wikipedia.org/wiki/Cpe 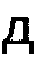 a_LB). Dieses Medium enthält Peptide und Peptone, Vitamine und Spurenelemente. Die Quelle der Peptide und Peptone ist Trypton. Die Vitamine und Spurenelemente sind im Hefeextrakt enthalten und die Natriumionen, die für den Transport und das osmotische Gleichgewicht notwendig sind, werden dem Medium in Form von Natriumchlorid zugesetzt.

Für die Herstellung von 1 Liter LB-Medium werden 10g Trypton, 5g Hefeextrakt und 10g NaCL gemischt. Die gewonnene Mischung wird in destilliertem Wasser aufgelöst und das Endvolumen auf 1 Liter gebracht wird. Die gewonnene Lösung verbleibt im Autoklaven bei 120°C für 20 Minuten.

Dieses Nährmedium ist wirtschaftlich akzeptabler, enthält aber immer noch teure und seltene Inhaltstoffe wie Trypton und Hefeextrakt.

Das Nährmedium (Patent RU Nr. 2022008) auf der Basis von wässriger fermentierter Geflügelkotlösung kommt der Erfindung und ihrem technischen Wesen am nächsten. Die Basis des Nährmediums ist eine flüssige Fraktion des Geflügelkots, die vorher und nachher einer aeroben Behandlung unterzogen wird, wobei die vorherige aerobe Fermentation bei einer Temperatur von 28-40°C für 48 Stunden, und die nachfolgende aerobe Fermentation bei einer Temperatur von 60°C für 3-5 Tage durchgeführt wird. Das resultierende Gemisch wird zentrifugiert, die überstehende Fraktion abgetrennt und sterilisiert.

Der Nachteil dieses Nährmediums ist sein trotz der Verfügbarkeit des Ausgangsstoffes (Geflügelkot) arbeits- und energieintensiver Herstellungsprozess. Die Dauer des Prozesses beträgt 5 bis 7 Tage, während der die für die Fermentationsreaktion erforderliche Temperatur aufrechterhalten werden muss. Außerdem können im Ausgangsrohstoff (Geflügelkot) Spuren von Antibiotika, Schwermetallen und anderen Schadstoffen enthalten sein. Des Weiteren benötigt dieses Medium bestimmte Lagerbedingungen.

Das Ziel der vorliegenden Erfindung ist die Beseitigung der oben genannten Nachteile von Nährmedien und die Herstellung eines hochwertigen bakteriellen Biopräparates, das als Dünger für die Blatt- und Wurzeldüngung bei Pflanzen, als Fungizid, als Futtermittelzusatz für Tiere oder Vögel, als Biopräparat zur Förderung der Methanogenese bei der Biogasproduktion und als Stimulator für Pflanzenwachstum verwendet werden kann.

Das genannte Ziel wird durch das bakterielle Biopräparat und das Verfahren seiner Herstellung erreicht, das gemäß der vorliegenden Erfindung offenbart wird.

Das bakterielle Biopräparat gemäß der vorliegenden Erfindung enthält eine ausreichende Menge an Mikroorganismen, wird durch ein kostengünstiges Verfahren hergestellt, erfordert keine besonderen Lagerbedingungen und hat zudem eine lange Lagerzeit.

Der erste Erfindungsgegenstand ist ein bakterielles Biopräparat, das Mikroorganismen der Gattungen *Bacillus, Azotobacter, Cellulomonas* und *Pseudomonas* und ein Nährmedium für Bakterien enthält, das ein alkalisches Extrakt aus organischen Substanzen pflanzlichen oder natürlichen Ursprungs aus Kaustobiolithen ist. Außerdem kann dieses Medium zusätzlich, optional, Spurenelemente enthalten.

In einer bevorzugten Ausführungsform der Erfindung enthält das Biopräparat zusätzlich Mikroorganismen der Gattung ausgewählt aus der Gruppe enthaltend *Agrobacterium, Azospizillum, Amonas, Cellulosimicrobiom, Geobacillus, Rhizobium, Arthrobacter, Verticillium, Beijerinckia, Lactobacillus, Cytophaga, Archangium, Sorangium, Polimiqsa, Cellvibrio, Clostridium, Micromonosporzoachalcea, Streptomyces, Cellulosea, Stpeptosporangium, Trichoderma, Aspergilus* und Kombinationen davon.

Mikroorganismen der Gattungen *Bacillus, Azotobacter, Cellulomonas, PseudomonasAgrobacterium, Azospizillum, Amonas, Cellulosimicrobiom, Geobacillus, Rhizobium, Arthrobacter, Verticillium, Beijerinckia, Lactobacillus, Cytophaga, Archangium, Sorangium, Polimiqsa, Cellvibrio, Clostridium, Micromonosporzoachalcea, Streptomyces, Cellulosea, Stpeptosporangium, Trichoderma, Aspergilus* sind kommerziell verfügbar.

In einer weiteren bevorzugten Ausführungsform der Erfindung beträgt der Trockensubstanzgehalt im Biopräparat 10,0 bis 20,0 Gew.-%, vorzugsweise 15,0 Gew.-%, und der Gehalt an organischen Substanzen im Biopräparat beträgt 2,5 bis 15,0 Gew.-%, vorzugsweise 12,5 Gew.-%.

In einer weiteren bevorzugten Ausführungsform der Erfindung sind die im Biopräparat enthaltenen organischen Substanzen Huminsäuren, Fulvosäuren und Aminosäuren. Die Huminsäuren sind in einer Menge von 3,0 bis 7,0 Gew.-%, vorzugsweise 5,0 Gew.-%, die Fulvosäuren in einer Menge von 2,0 bis 5,0 Gew.-%, vorzugsweise 3,0 Gew.-%, die Aminosäuren in einer Menge von 3,0 bis 6,0 Gew.-%, vorzugsweise 4,5 Gew.-%, enthalten.

In einer weiteren bevorzugten Ausführungsform der Erfindung enthält das Biopräparat zusätzlich Makroelemente ausgewählt aus der Gruppe enthaltend Stickstoff, Phosphor, Kalium, Calcium, Magnesium und Kombinationen davon. In einer besonders bevorzugten Ausführungsform der Erfindung sind die Makroelemente im Biopräparat in den folgenden Mengen enthalten: 0,5 bis 3,0 Gew.-%, vorzugsweise 1,5 Gew.-%, Stickstoff; 0,5 Gew.-% oder weniger, vorzugsweise 0,01 Gew.-%, Phosphor; 4,0 bis 8,0 Gew.-%, vorzugsweise 6,0 Gew.-%, Kalium; 0,02 bis 0,1 Gew.-%, vorzugsweise 0,05 Gew.-%, Magnesium.

In einer weiteren bevorzugten Ausführungsform der Erfindung enthält das Biopräparat zusätzlich, optional Spurenelemente ausgewählt aus der Gruppe enthaltend Kupfer, Zink, Kobalt, Molybdän, Mangan, Jod, Bor, Selen und Kombinationen davon. In einer besonders bevorzugten Ausführungsform der Erfindung sind die Spurenelemente in dem Biopräparat in den folgenden Mengen enthalten: 10 bis 20 mg/kg, vorzugsweise 15 mg/kg, Kupfer; 100 bis 130 mg/kg, vorzugsweise 120 mg/kg, Zink; 1,5 bis 3,0 mg/kg, vorzugsweise 2,17 mg/kg, Kobalt; 15 bis 25 mg/kg, vorzugsweise 20 mg/kg, Molybdän; 15 bis 25 mg/kg, vorzugsweise 20 mg/kg, Mangan; und 5 bis 15 mg/kg, vorzugsweise 10 mg/kg, Selen.

Der zweite Erfindungsgegenstand ist ein Verfahren zur Herstellung eines bakteriellen Biopräparates gemäß der Erfindung, das die Durchführung der folgenden Schritte umfasst:
1) Mahlen der Kaustobiolithen und Mischen des erhaltenen Rohstoffes mit Wasser,
2) Extrahieren der organischen Substanzen aus der in Schritt 1) erhaltenen Mischung durch Zugabe von Kaliumhydroxid zu dieser Mischung,
3) Neutralisieren der Extraktionsreaktion aus Schritt (2) durch Zugabe von oxidierter Braunkohle oder Torf, wodurch das Nährmedium für die Vermehrung von Mikroorganismen erhalten wird, und
4) Einführen des in Schritt 3) erhaltenen Nährmediums und der Mischung von Mikroorganismen in den Bioreaktor und Erhalt eines flüssigen bakteriellen Biopräparates durch Inkubation.

In einer bevorzugten Ausführungsform der Erfindung sind die Kaustobiolithe ausgewählt aus der Gruppe enthaltend Torf, fossile Kohle, Schiefer, Schungit und Kombinationen davon.

In einer weiteren bevorzugten Ausführungsform des erfindungsgemäßen Verfahrens werden die Kaustobiolithe auf Partikelgrößen von 1 bis 200µm gemahlen.

In einer weiteren bevorzugten Ausführungsform des erfindungsgemäßen Verfahrens beträgt das Verhältnis der gemahlenen Kaustobiolithen und Wasser in Schritt 1) 1:5 (Kaustobiolithe zu Wasser).

In einer weiteren bevorzugten Ausführungsform des erfindungsgemäßen Verfahrens wird die Extraktion in Schritt 2) in einem Rührwerk für 3 Stunden bei einer Temperatur von 80°C und einer Rührdrehzahl von 90 U/min durchgeführt. Des Weiteren wird Kaliumhydroxid zugegeben, bis ein pH-Wert von 9 erreicht ist.

In einer weiteren bevorzugten Ausführungsform des erfindungsgemäßen Verfahrens wird die Braunkohle oder der Torf in Schritt 3) zugegeben bis der pH-Wert 7,8 beträgt. In der am meisten bevorzugten Ausführungsform wird die Neutralisation in Schritt 3) durch Zugabe von oxidierter Braunkohle durchgeführt.

In einer weiteren bevorzugten Ausführungsform des erfindungsgemäßen Verfahrens wird die Neutralisation in Schritt (3) 2 Stunden lang durchgeführt.

In einer weiteren bevorzugten Ausführungsform des erfindungsgemäßen Verfahrens werden dem in Schritt (3) erhaltenen Nährmedium zusätzlich Spurenelemente ausgewählt aus der Gruppe enthaltend Kupfer, Zink, Kobalt, Molybdän, Mangan, Jod, Bor, Selen und Kombinationen davon zugesetzt.

In einer weiteren bevorzugten Ausführungsform des erfindungsgemäßen Verfahrens kann die Mikroorganismenmischung, die in Schritt (4) eingeführt wird, durch Vermehrung der Mikroorganismen auf einem Substrat, das ein für ihre Vermehrung geeignetes Nährmedium enthält, vorproduziert werden.

In einer weiteren bevorzugten Ausführungsform des erfindungsgemäßen Verfahrens wird die Inkubation der Mikroorganismen in Schritt 4) bei einer Temperatur von 28 bis 32°C, bei einer Rührdrehzahl des Bioreaktors von 180U/min und bei einer Luftzufuhr von 1 Liter Luft pro 1 Liter des in den Reaktor zugeführten Nährmittels für 48 Stunden durchgeführt.

In einer weiteren bevorzugten Ausführungsform des erfindungsgemäßen Verfahrens wird das in Schritt 4) erhaltene flüssige bakterielle Biopräparat zur Lagerung und/ oder zum Transport abgefüllt.

In einer weiteren bevorzugten Ausführungsform des erfindungsgemäßen Verfahrens wird das in Schritt 4) erhaltene flüssige bakterielle Biopräparat einer Verdampfung unter Vakuum oder in einem lyophilen Trockner unterzogen, um ein pastöses oder pulverförmiges Biopräparat zu erhalten.

Der dritte Erfindungsgegenstand ist die Verwendung des bakteriellen Biopräparates als Düngemittel für die Blatt- und Wurzelerdüngung von Pflanzen, oder als Stimulator für das Pflanzenwachstum, oder als Fungizid, oder als Futtermittelzusatz für Tiere oder Vögel, oder als Präparat zur Förderung der Methanogenese bei der Biogaserzeugung.

Bei den obengenannten erfindungsgemäßen Verwendungsgegenständen scheiden die Mikroorganismen des bakteriellen Biopräparates, die sich von den im Nährmedium enthaltenen organischen Substanzen ernähren, nützliche Metaboliten aus, die in verschiedenen Bereichen erfolgreich eingesetzt werden.

Insbesondere in den obengenannten erfindungsgemäßen Verwendungen:
a) in dem Biopräparat als Stimulator für das Pflanzenwachstum scheiden die Mikroorganismen, die sich vom im Biopräparat enthaltenen Nährmedium ernähren, den Stimulator Heteroauxin, Vitamine und verschiedene organische Säuren aus;
b) in dem Biopräparat als Düngemittel entwickeln sich Mikroorganismen, die sich vom im Biopräparat enthaltenen Nährmedium ernähren und die Zellulose, organische und mineralische Phosphorverbindungen aufbrechen, Harnstoff und Kaliumsilikate spalten und Phosphor mobilisieren;
c) in dem Biopräparat als Futtermittelzusatz scheiden Mikroorganismen, die sich von den im Biopräparat enthaltenen Nährböden ernähren, Präbiotika, Vitamine, Enzyme und antimikrobielle Verbindungen aus;
d) in dem Biopräparat als Präparat zur Förderung der Methanogenese in der Biogasproduktion entwickeln sich fakultative Mikroorganismen, die zum Abbau von Zellulose beitragen und die Enzyme Cellulase, Protease, Amylase und Lipase bilden, welche Katalysatoren in der Methanproduktion sind.

Das bakterielle Biopräparat gemäß der vorliegenden Erfindung kann bei einer Reihe von landwirtschaftlichen Kulturen angewendet werden. Solche Kulturen können insbesondere Getreide, Hülsenfrüchte, Nutzpflanzen, Ölsaaten, Hackfrüchte, Faserpflanzen, Beerenkulturen, Obstbäume, Ziersträucher und Rasengräser sein, wenn das Biopräparat in offenen Böden angewendet werden soll. Bei bedeckten Böden handelt es sich bei den Pflanzen insbesondere um Gemüsepflanzen, Hülsenfrüchte, Hackfrüchte oder im Pflanzenbau kultivierte Blühpflanzen.

Das Biopräparat kann durch Bodenausbringung, Behandlung von Samen oder Saatgut vor der Aussaat sowie Blattbehandlung von Pflanzen während des Wachstums angewendet werden.

Die im Folgenden dargestellten Beispiele beschreiben einige Ausführungsformen der Erfindung, sollen diese aber nicht einschränken.

### Beispiel 1.

Das Rohmaterial, das Kaustobiolithe enthält wird auf eine Partikelgröße von 1 bis 200µm gemahlen und mit Wasser in einem Reaktor gemischt, bis ein Verhältnis von Kaustobiolithen zu Wasser von 1:5 erreicht ist. Zu der erhaltenen Mischung wird Kaliumhydroxid zugegeben bis ein pH-Wert von 9 erreicht ist. Die Extraktion wird innerhalb von 3 Stunden bei einer Temperatur von 80°C und einer Rührdrehzahl von 90 U/min durchgeführt.

Anschließend wird 2 Stunden lang eine Neutralisation durch Zugabe von oxidierter Braunkohle zum Extraktionsgemisch durchgeführt, bis der pH-Wert des Gemischs 7,8 beträgt.

Spurenelemente wie Kupfer, Zink, Kobalt, Molybdän, Mangan, Jod, Bor und Selen werden in den Bioreaktor gegeben, und das Rühren wird für 1 Stunde fortgesetzt mit nachfolgender Reaktorkühlung.

Das so erhaltene flüssige Extrakt aus Kaustobiolithen ist ein hervorragender heterotropher Nährrohstoff für die Lebenstätigkeit saprophytärer Mikroorganismen. Die Konzentration der organischen Substanzen im erhaltenen Nährmedium liegt zwischen 2,5 und 15,0 Gew.-%. Die bevorzugte Menge organischer Substanzen beträgt 12,5 Gew.-%.

Der vergleichbare Nutzeffekt des im Biopräparat gemäß der vorliegenden Erfindung enthaltenen Nährmediums und der aus dem Stand der Technik bekannten Nährmedien ist in Tabelle 1 dargestellt.

**Tabelle 1.**

| Anzahl der Mikroorganismen bei unterschiedlichen Konzentrationen organischer Substanzen in einem Extrakt aus Kaustobiolithen und in Nährmedien auf Basis von Fleisch-Pepton-Agar (FPA) und Luria | | | | | | | | | |
|---|---|---|---|---|---|---|---|---|---|
| Bezeichnung des Bakterienstammes | | Anzahl der Mikroorganismen 10¹² auf 1 mg des Mediums | | | | | | | |
| | | Gehalt an organischen Substanzen im Nährmedium auf Basis eines Extraktes aus Kaustobiolithen in Gew.-% | | | | | | Medium FPA | Medium Luria |
| | | 15% | 12,5% | 10% | 7,5% | 5% | 2,5% | | |
| Bacillus megaterium | | | | | | | | | |
| Stamm 1 | | 876 | 966 | 471 | 1286 | 652 | 1975 | 812 | 684 |
| Stamm 2 | | 916 | 1464 | 564 | 80 | 1088 | 286 | 1304 | 852 |
| Stamm 3 | | 1664 | 1522 | 1272 | 944 | 1016 | 1026 | 1466 | 984 |
| Bacillus mucilaginosus | | | | | | | | | |
| Stamm 1 | | 1568 | 2456 | 1312 | 1896 | 397 | 824 | 1264 | 1136 |
| Stamm 2 | | 532 | 1277 | 198 | 1024 | 257 | 493 | 1677 | 399 |
| Bacilus Sp | | | | | | | | | |
| Stamm 1 | | 1156 | 2136 | 1430 | 624 | 121 | 672 | 1041 | 990 |
| Stamm 2 | | 1424 | 2368 | 3508 | 4444 | 3224 | 1732 | 2560 | 2400 |
| Rizobium radiobacter | | | | | | | | | |
| Stamm 1 | | 3600 | 4992 | 2268 | 4879 | 3325 | 2553 | 4826 | 4220 |
| Stamm 2 | | 3971 | 4452 | 3707 | 3310 | 4804 | 3854 | 3664 | 3817 |
| Cellulomonas uda | | 2352 | 3368 | 2460 | 1132 | 439 | 1656 | 2616 | 2744 |
| Cellulomonas sp | | 3496 | 4445 | 1925 | 6956 | 3672 | 2586 | 4501 | 2968 |
| Artrobacter sp | | 2724 | 2608 | 3688 | 1240 | 3640 | 3795 | 3448 | 2120 |
| Bacillus lichinoformis | | | | | | | | | |
| Stamm 1 | | 2358 | 2704 | 1888 | 1802 | 1128 | 2004 | 2444 | 2152 |
| Stamm 2 | | 2324 | 2287 | 2356 | 1496 | 1072 | 2560 | 2336 | 2584 |
| Stamm 3 | | 1752 | 2928 | 2208 | 1484 | 3592 | 4456 | 2072 | 2312 |
| Bacillus atropheus | | 2101 | 3122 | 1238 | 1441 | 2012 | 632 | 1608 | 2311 |
| Pseudomonas cedrina | | 2864 | 3022 | 1700 | 1913 | 1568 | 1600 | 2604 | 3128 |
| Pseudomonas sp | | 1414 | 2132 | 1552 | 1302 | 1368 | 2576 | 1616 | 2008 |
| Pseudomonas putida | | 2348 | 2956 | 4800 | 1020 | 1376 | 1624 | 2624 | 1904 |
| Bacillus subtilis | | | | | | | | | |
| Stamm 1 | | 1631 | 2708 | 1003 | 1864 | 4467 | 1243 | 2472 | 2279 |
| Stamm 2 | | 3694 | 3810 | 5511 | 2851 | 3562 | 3827 | 2607 | 2415 |
| Stamm 3 | | 2502 | 2600 | 1572 | 1552 | 1176 | 780 | 1880 | 2271 |
| Stamm 4 | | 2431 | 3346 | 3008 | 9718 | 1544 | 3869 | 2049 | 2666 |
| Stamm 5 | | 2431 | 3346 | 3008 | 9718 | 1544 | 3869 | 2049 | 2666 |
| Stamm 6 | | 2214 | 2061 | 4060 | 4414 | 1076 | 3697 | 1653 | 1075 |
| Stamm 7 | | 2490 | 4112 | 2183 | 1286 | 3424 | 4414 | 8828 | 3597 |
| Stamm 8 | | 1424 | 2730 | 4114 | 2266 | 1956 | 2821 | 1435 | 2150 |

Ein Beispiel für die chemische Zusammensetzung des flüssigen Biopräparates gemäß der vorliegenden Erfindung ist in der folgenden Tabelle 2 angeführt.

**Tabelle 2**

| Chemische Zusammensetzung des flüssigen Biopräparates | |
|---|---|
| Trockensubstanz | 15 Gew.% |
| Organische Substanz | 10 Gew.-% |
| Stickstoff | 1,5 Gew.-% |
| Kalium | 6 Gew.-% |
| Phosphor | <0,01 Gew.-% |
| Magnesium | 0,05 Gew.-% |
| Kobalt | 2,17mg/kg |
| Kupfer | 15 mg/kg |
| Mangan | 20 mg/kg |
| Molybdän | 20 mg/kg |
| Selen | 10 mg/kg |
| Zink | 120 mg/kg |

Die in dem Biopräparat enthaltene Mischung von Mikroorganismen ist vorzugsweise eine Mischung von Bakterien der Gattung Baccillus, vorzugsweise in einer Menge von nicht weniger als 1×10⁹KBE/ml Kulturflüssigkeit; Gattung Cellulomonac, vorzugsweise in einer Menge von nicht weniger als 1×10⁹ KBE/ml Kulturflüssigkeit; Gattung Pseudomonas, vorzugsweise in einer Menge von nicht weniger als 1×10⁹ KBE/ml Kulturflüssigkeit.

### Beispiel 2

Beispiel 2 wird wie Beispiel 1 ausgeführt, jedoch wird oxidierter Torf zur Neutralisation der Extraktionsreaktion verwendet.

### Beispiel 3

Die Ergebnisse der Analyse des Biopräparates in Abhängigkeit von seiner Lagerzeit sind in Tabelle 3 dargestellt. Das Biopräparat wurde für 2 Jahre unter Standardlagerbedingungen bei -4 bis +35°C gelagert.

**Tabelle 3**

| Anzahl der Mikroorganismen im Biopräparat in Abhängigkeit von der Lagerzeit in naten. | | | | | |
|---|---|---|---|---|---|
| Bezeichnung des Bakterienstammes | Anzahl der Mikroorganismen 10¹² pro 1 mg Medium | | | | |
| | Lagerdauer des Biopräparates in Monaten | | | | |
| | 0,3 | 6 | 12 | 18 | 24 |
| Bacillus megaterium | | | | | |
| Stamm 1 | 966 | 712 | 450 | 74 | 13 |
| Stamm 2 | 1464 | 815 | 615 | 89 | 49 |
| Stamm 3 | 1522 | 778 | 521 | 105 | 65 |
| Bacillus mucilaginosus | | | | | |
| Stamm 1 | 2456 | 1500 | 921 | 64 | 27 |
| Stamm 2 | 1277 | 613 | 481 | 71 | 41 |
| Bacilus Sp | | | | | |
| Stamm 1 | 2136 | 1511 | 1041 | 82 | 47 |
| Stamm 2 | 2368 | 1291 | 415 | 37 | 25 |
| Rizobium radiobacter | | | | | |
| Stamm 1 | 4992 | 2114 | 721 | 50 | 34 |
| Stamm 2 | 4452 | 1701 | 609 | 49 | 57 |
| Cellulomonas uda | 3368 | 1918 | 915 | 39 | 28 |
| Cellulomonas sp | 4445 | 2251 | 418 | 44 | 29 |
| Artrobacter sp | 2608 | 2109 | 431 | 27 | 38 |
| Bacillus lichinoformis | | | | | |
| Stamm 1 | 2704 | 1803 | 251 | 94 | 79 |
| Stamm 2 | 2287 | 1914 | 123 | 89 | 15 |
| Stamm 3 | 2928 | 1151 | 414 | 105 | 37 |
| Bacillus atropheus | 3122 | 1915 | 322 | 94 | 66 |
| Bseudomonas cedrina | 3022 | 1211 | 180 | 52 | 19 |
| Bseudomonas sp | 2132 | 1050 | 488 | 39 | 17 |
| Bseudomonas putida | 2956 | 1411 | 315 | 40 | 37 |
| Bacillus subtilis | | | | | |
| Stamm 1 | 2708 | 1913 | 413 | 23 | 12 |
| Stamm 2 | 3810 | 1916 | 214 | 71 | 31 |
| Stamm 3 | 2600 | 1117 | 321 | 56 | 72 |
| Stamm 4 | 3346 | 1212 | 134 | 51 | 56 |
| Stamm 5 | 3346 | 1714 | 225 | 78 | 45 |
| Stamm 6 | 2061 | 1321 | 578 | 54 | 34 |
| Stamm 7 | 4112 | 1619 | 313 | 61 | 72 |
| Stamm 8 | 2730 | 1113 | 219 | 94 | 82 |

Die Ergebnisse zeigen, dass die bevorzugte Lagerzeit des Biopräparates bis zu 6 Monaten beträgt. Gleichzeitig behält das Biopräparat seine Eigenschaften bei einer Lagerzeit von bis zu 12 Monaten bei -4 bis +35°C.

Das so erhaltene bakterielle Biopräparat enthält eine ausreichende Menge an Mikroorganismen, wird durch ein kostengünstiges Verfahren hergestellt, erfordert keine besonderen Lagerbedingungen und hat zudem eine lange Lagerzeit.

## Patentansprüche

1. Ein bakterielles Biopräparat, enthaltend Mikroorganismen der Gattungen *Bacillus, Azotobacter, Cellulomonas* und *Pseudomonas* und ein Nährmedium für Bakterien, das ein alkalischer Extrakt aus organischen Substanzen pflanzlichen oder natürlichen Ursprungs aus Kaustobiolithen ist.

2. Biopräparat nach Anspruch 1, zusätzlich enthaltend Mikroorganismen einer Gattung, ausgewählt aus der Gruppe enthaltend *Agrobacterium, Azospizillum, Amonas, Cellulosimicrobiome, Geobacillus, Rhizobium, Arthrobacter, Verticillium, Beijerinckia, Lactobacillus, Cytophaga, Archangium, Sorangium, Polimiqsa, Cellvibrio, Clostridium, Micromonosporzoachalcea, Streptomyces, Cellulosea, Stpeptosporangium, Trichoderma, Aspergilus* und deren Kombinationen.

3. Biopräparat nach Anspruch 1 oder 2, wobei der Gehalt an Trockensubstanz von 10,0 bis 20,0 Gew.-%, vorzugsweise 15,0 Gew.-%, und der Gehalt an organischen Substanzen von 2,5 bis 15,0 Gew.-%, vorzugsweise 12,5 Gew.-%, beträgt.

4. Biopräparat nach Anspruch 3, wobei die organischen Substanzen Huminsäuren, Fulvosäuren und Aminosäuren sind, wobei
der Huminsäuregehalt 3,0 bis 7,0 Gew.-%, vorzugsweise 5,0 Gew.-% beträgt,
der Fulvosäuregehalt 2,0 bis 5,0 Gew.-%, vorzugsweise 3,0 Gew.-% beträgt, und
der Aminosäuregehalt 3,0 bis 6,0 Gew.-%, vorzugsweise 4,5 Gew.-% beträgt.

5. Biopräparat nach einem der Ansprüche 1 bis 4, zusätzlich umfassend Makroelemente, ausgewählt aus der Gruppe enthaltend Stickstoff, Phosphor, Kalium, Calcium, Magnesium und Kombinationen davon.

6. Biopräparat nach Anspruch 5, wobei die Makroelemente in den folgenden Mengen enthalten sind:
0,5 bis 3,0 Gew.-%, vorzugsweise 1,5 Gew.-%, Stickstoff;
0,5 Gew.-% oder weniger, vorzugsweise 0,01 Gew.-%, Phosphor;
4,0 bis 8,0 Gew.-%, vorzugsweise 6,0 Gew.-%, Kalium; und
0,02 bis 0,1 Gew.-%, vorzugsweise 0,05 Gew.-%, Magnesium.

7. Biopräparat nach einem der Ansprüche 1 bis 6, zusätzlich enthaltend Spurenelemente, ausgewählt aus der Gruppe enthaltend Kupfer, Zink, Kobalt, Molybdän, Mangan, Jod, Bor, Selen und Kombinationen davon.

8. Verfahren zur Herstellung eines bakteriellen Biopräparates nach einem der Ansprüche 1 bis 7 wobei:
1) die Kaustobiolithe zerkleinert werden und der erhaltene Rohstoff mit Wasser vermischt wird,
2) die organischen Substanzen aus der in Schritt (1) erhaltenen Mischung durch Zugabe von Kaliumhydroxid zu dieser Mischung extrahiert werden,
3) die Extraktionsreaktion aus Schritt (2) durch Zugabe von oxidierter Braunkohle oder Torf neutralisiert wird, wodurch ein Nährmedium für die Vermehrung von Mikroorganismen erhalten wird, und
4) das in Schritt 3) erhaltene Nährmedium und die Mikroorganismenmischung in den Bioreaktor eingeführt werden und ein flüssiges bakterielles Biopräparat durch Inkubation erhalten wird.

9. Verfahren nach Anspruch 8, wobei Kaustobiolithe ausgewählt aus der Gruppe umfassend Sapropel, Torf, fossile Kohle, Schiefer, Schungit und eine Kombination davon verwendet werden.

10. Verfahren nach Anspruch 8 oder 9, wobei die verwendeten Kaustobiolithe auf eine Partikelgröße von 1 bis 200 µm gemahlen werden.

11. Verfahren nach einem der Ansprüche 8 bis 10, wobei das Mischen der Kaustobiolithe mit Wasser in Schritt (1) durchgeführt wird, bis das Verhältnis von Kaustobiolithen zu Wasser 1:5 beträgt.

12. Verfahren nach einem der Ansprüche 8 bis 11, wobei das Kaliumhydroxid in Schritt (2) zugegeben wird, bis ein pH-Wert der Mischung von 9 erreicht ist.

13. Verfahren nach einem der Ansprüche 8 bis 12, wobei die Extraktion in Schritt (2) in einem Rührwerk für 3 Stunden bei einer Temperatur von 80°C und einer Rührdrehzahl von 90 U/min durchgeführt wird.

14. Verfahren nach einem der Ansprüche 8 bis 13, wobei Braunkohle oder Torf in Schritt (3) zugegeben wird, bis ein pH-Wert der Mischung von 7,8 erreicht ist.

15. Verfahren nach einem der Ansprüche 8 bis 14, wobei die Neutralisation in Schritt (3) durch Zugabe von oxidierter Braunkohle durchgeführt wird.

16. Verfahren nach einem der Ansprüche 8 bis 15, wobei die Neutralisation in Schritt (3) über einem Zeitraum von 2 Stunden durchgeführt wird.

17. Verfahren nach einem der Ansprüche 8 bis 16, wobei dem in Schritt (3) erhaltenen Nährmedium zusätzlich Spurenelemente ausgewählt aus der Gruppe umfassend Kupfer, Zink, Kobalt, Molybdän, Mangan, Iod, Bor, Selen und Kombinationen davon zugesetzt werden.

18. Verfahren nach einem der Ansprüche 8 bis 17, wobei die Inkubation der Mikroorganismen in Schritt (4) bei einer Temperatur von 28 bis 32°C, bei einer Rührdrehzahl im Bioreaktors von 180 U/min und bei einer Luftzufuhr von 1 Liter Luft pro 1 Liter in den Reaktor geladenem Nährmedium für 48 Stunden durchgeführt wird.

19. Verfahren nach einem der Ansprüche 8 bis 18, zusätzlich umfassend einen Schritt, bei dem das in Schritt (4) erhaltene flüssige bakterielle Biopräparat einer Verdampfung unter Vakuum oder in einem lyophilen Trockner unterzogen wird, um ein pastöses oder pulverförmiges Biopräparat zu erhalten.

20. Verwendung des bakteriellen Biopräparates nach einem der Ansprüche 1 bis 7 als Düngemittel zur Blatt- und Wurzeldüngung von Pflanzen, oder als Stimulator des Pflanzenwachstums, oder als Fungizid, oder als Futtermittelzusatz für Tiere und Vögel, oder als Biopräparat zur Förderung der Methanogenese bei der Biogasproduktion.
